# EUROPEAN PATENT APPLICATION

(11) **EP 4 575 454 A1**
(43) Date of publication of application: **25.06.2025**
(21) Application number: 23891280.2
(22) Date of filing: 19.10.2023
(51) Int. Cl.: G01N 1/28, G01N 1/32, G01N 33/204

(54) **STEEL ETCHING METHOD, METHOD FOR PREPARING SAMPLE FOR OPTICAL MICROSCOPE OBSERVATION, STEEL ETCHING SOLUTION SET, AND ETCHING DEVICE**

(30) Priority: 17.11.2022 JP 2022183805
(71) Applicant: JFE Steel Corporation, Tokyo 100-0011 (JP)
(72) Inventor: KAMO, Yuichi, Tokyo 100-0011 (JP)
(74) Representative: Haseltine Lake Kempner LLP
(86) International application number: PCT/JP2023/037790
(87) International publication number: WO 2024/106124

(57) **Abstract**

Provided are a method for etching steel, a method for preparing a sample for optical microscopic observation, a solution set for etching steel, and an etching apparatus with which it is possible to analyze a microstructure with ease and high accuracy.

A method for etching steel, the method including a first etching process of performing electrolytic etching on steel in a basic solution having a pH of more than 7.0 and a second etching process of bringing the steel, which has been subjected to the first etching process, into contact with an acidic solution having a pH of less than 7.0. The basic solution may have a pH of 13.0 or more and may be a KOH aqueous solution. The electrolytic etching may be performed with an electrical current density of 0.5 A/cm² or more. The steel may have a microstructure including martensite and ferrite.

## Description

### Technical Field

The present invention relates to a method for etching, a method for preparing a sample for optical microscopic observation, a solution set for etching steel, and an etching apparatus with which, when the microstructure of steel including a martensite phase and a ferrite phase or the like is observed, it is possible to identify with high accuracy the fractions of the phases which have effects on material properties by performing image analysis on an optical micrograph.

### Background Art

Oil wells in harsh corrosive environments such as environments containing carbon dioxide gas and deep oil fields are being actively developed. Examples of steel pipes for oil wells usable in such environments include stainless steel pipes having microstructures including martensite and ferrite which are proposed in Patent Literature 1 and Patent Literature 2.

### Citation List

### Patent Literature

PTL 1: Japanese Patent No. 5348354
PTL 2: International Publication No. 2017/010036

### Summary of Invention

### Technical Problem

The stainless steels described above have microstructures including martensite and ferrite, and the characteristics of the microstructure such as phase fractions have effects on the properties of the steel. There is a case where the phase fractions of a manufactured steel pipe are determined for quality control, and it is required that the phase fraction determination be performed on steel pipes manufactured in large quantities with not only high accuracy but also ease.

Incidentally, the stainless steels disclosed in Patent Literature 1 and Patent Literature 2 have microstructures including martensite, ferrite, and retained austenite. Of such phases, it is comparatively easy for the phase fraction of retained austenite to be determined by using X-ray diffractometry. On the other hand, regarding the distinction between martensite and ferrite, in Patent Literature 1, after the stainless steel has been polished, the polished stainless steel is etched in a mixture solution of aqua regia and glycerin, an optical micrograph is taken at a magnification of 100 times, and the ferrite phase fraction is determined on the micrograph by using a point counting method in accordance with JIS G 0555. In the determination utilizing a point counting method, a glass plate having 20 vertical and 20 horizontal grid lines is inserted into the eyepiece of a microscope, and the number of grid point centers covered by the relevant phase (ferrite) is counted. In addition, it is prescribed that at least 30 fields of view be observed per specimen. Therefore, determining the ferrite phase fraction of one specimen is time consuming. In addition, regarding the distinction between martensite and ferrite, in Patent Literature 2, after etching has been performed in Vilella's reagent (mixture solution of 100 mL of ethanol, 10 mL of hydrochloric acid, and 2 g of picric acid), the image of the steel microstructure is taken by using a scanning electron microscope, and the ferrite phase fraction is determined by using an image analyzer. This method requires an expensive analyzer, and there is a problem in that investigating a large quantity of specimens is time consuming because the images are taken under vacuum.

With reference to the Patent Literature described above, examples of an easy method for investigating the phase fractions of martensite and ferrite include one in which an optical microscope is used to take images and one in which an image analyzer is used to determine quantity. When investigations were conducted regarding a method in which a sample is prepared by performing etching by using the etching method described in Patent Literature 1 or Patent Literature 2, that is, by using the mixture solution of aqua regia and glycerin or Vilella's reagent, an optical micrograph of the etched sample is taken, and image analysis is performed, it was found that there is a problem from the viewpoint of accuracy. Specifically, since martensite is etched when one of the etching methods described above is used, martensite has a dark appearance and ferrite has a light appearance in an optical micrograph. However, not the whole surface of martensite has a dark appearance, and, since there is a contrast variation in accordance with crystal orientation, a portion of martensite tends to have an appearance close to that of ferrite. Therefore, when image analysis is performed, a portion which should essentially be identified as martensite tends to be incorrectly identified as ferrite, which may result in a deterioration in the accuracy of determining the ferrite phase fraction.

As described above, with respect to the microstructure of steel having a microstructure including martensite and ferrite or the like, there has been a demand for a technique with which it is possible to distinguish phases from each other with ease and higher accuracy.

The present invention has been completed to solve the problems described above, and an object of the present invention is to provide a method for etching steel, a method for preparing a sample for optical microscopic observation, a solution set for etching steel, and an etching apparatus with which it is possible to analyze a microstructure with ease and high accuracy.

### Solution to Problem

To solve the problems described above, the present inventors diligently conducted investigations regarding a method for etching stainless steel having a microstructure including martensite and ferrite as an example of a method for etching steel and, as a result, found that, by first performing electrolytic etching utilizing, for example, a KOH aqueous solution and by thereafter performing etching utilizing an acid, martensite is uniformly etched and there is an increased contrast difference with respect to ferrite.

The present invention has been completed on the basis of the knowledge described above and additional investigations. That is, the subject matter of the present invention is as follows.
[1] A method for etching steel, the method including
   a first etching process of performing electrolytic etching on steel in a basic solution having a pH of more than 7.0, and
   a second etching process of bringing the steel, which has been subjected to the first etching process, into contact with an acidic solution having a pH of less than 7.0.
[2] The method for etching steel according to item [1] above, in which the basic solution has a pH of 13.0 or more.
[3] The method for etching steel according to item [1] or [2] above, in which the basic solution is a KOH aqueous solution or a NaOH aqueous solution.
[4] The method for etching steel according to any one of items [1] to [3] above, in which the electrolytic etching is performed with an electrical current density of 0.5 A/cm² or more.
[5] The method for etching steel according to any one of items [1] to [4] above, in which the steel has a microstructure including martensite and ferrite.
[6] A method for preparing a sample for optical microscopic observation, in which the steel which has been obtained by using the method for etching steel according to any one of items [1] to [5] above is used as the sample for optical microscopic observation.
[7] A solution set for etching steel, the solution set including
   a basic solution having a pH of more than 7.0 for performing electrolytic etching on steel, and
   an acidic solution having a pH of less than 7.0 for performing etching on the steel.
[8] The solution set for etching steel according to item [7] above, in which the basic solution has a pH of 13.0 or more.
[9] The solution set for etching steel according to item [7] or [8] above, in which the basic solution is a KOH aqueous solution or a NaOH aqueous solution.
[10] The solution set for etching steel according to any one of items [7] to [9] above, in which the steel has a microstructure including martensite and ferrite.
[11] An etching apparatus including
   the solution set for etching steel according to any one of items [7] to [10] above, and
   electrodes.

### Advantageous Effects of Invention

According to the present invention, it is possible to analyze a microstructure with ease and high accuracy.

### Brief Description of Drawings

[Fig. 1] Fig. 1 shows one example of an optical micrograph of the microstructure of the sample obtained by using the etching method of Example 1 of the present invention.
[Fig. 2] Fig. 2 shows one example of an optical micrograph of the microstructure of the sample obtained by using the etching method of Comparative example 1.
[Fig. 3] Fig. 3 shows one example of an optical micrograph of the microstructure of the sample obtained by using the etching method of Comparative example 2.
[Fig. 4] Fig. 4 shows one example of an optical micrograph of the microstructure of the sample obtained by using the etching method of Example 2 of the present invention.

### Description of Embodiments

Hereafter, the embodiments of the present invention will be described.

The method for etching steel according to the present invention includes a first etching process of performing electrolytic etching on steel in a basic solution having a pH of more than 7.0 and a second etching process of bringing the steel, which has been subjected to the first etching process, into contact with an acidic solution having a pH of less than 7.0, thereby making it possible to perform analysis on the microstructure of the steel with ease and high accuracy in subsequent microscopic observation (optical microscopic observation).

There is no particular limitation on the steel which is etched in the present invention, and the microstructure of the steel may include martensite and ferrite. In addition, this steel may be stainless steel. The microstructure of the steel may include retained austenite. In the present invention, it is possible to distinguish with high accuracy between martensite and ferrite in the microstructure observation of steel which is performed by using an optical microscope or the like after etching has been performed.

### <First etching process>

In the first etching process, steel is subjected to electrolytic etching in a basic aqueous solution having a pH of more than 7.0. Specifically, electrolytic etching is performed on the steel by using an anodic polarization method in a basic solution having a pH of more than 7.0.

It is preferable that the basic solution have a pH of 13.0 or more. In addition, the pH in the present invention may be determined under the condition of a temperature of 25°C (hereinafter, also referred to as "pH(25°C)").

Here, in the present invention, the temperature of the solutions for performing etching (etching temperature) in the first etching process and the second etching process, which is described later, is not limited to 25°C.

In addition, there is no particular limitation on the basic solution, and examples of the basic solution include a KOH aqueous solution containing KOH and a NaOH aqueous solution containing NaOH.

The KOH aqueous solution is a solution containing KOH and water. The NaOH aqueous solution is a solution containing NaOH and water.

It is preferable that the KOH aqueous solution be an aqueous solution having a KOH concentration of 1.0 mass% or more. In addition, it is preferable that the NaOH aqueous solution be an aqueous solution having a NaOH concentration of 1.0 mass% or more.

### KOH concentration: 1.0 mass% or more, NaOH concentration: 1.0 mass% or more

By performing electrolytic etching in the KOH aqueous solution or the NaOH aqueous solution, martensite is likely to be more uniformly etched. To obtain such an effect, it is preferable that the KOH concentration in the KOH aqueous solution be 1.0 mass% or more. To obtain such an effect, it is preferable that the NaOH concentration in the NaOH aqueous solution be 1.0 mass% or more. It is more preferable that the concentration of the KOH aqueous solution be 10.0 mass% or more or even more preferably 20.0 mass% or more. It is more preferable that the concentration of the NaOH aqueous solution be 10.0 mass% or more or even more preferably 20.0 mass% or more. Although there is no particular limitation on the upper limit of the KOH concentration because it is important that KOH be contained, in the case where an excessive amount of KOH is added, since the effect becomes saturated because the KOH concentration reaches saturated KOH solubility, it is preferable that the concentration of the KOH aqueous solution be 50.0 mass% or less. It is also preferable that the concentration of the NaOH aqueous solution be 50.0 mass% or less.

### Electrical current density: 0.5 A/cm² or more

Since there is a variation in the degree of etching in accordance with the chemical composition of the steel, the electrical current density for performing electrolytic etching may be appropriately adjusted. However, it is preferable that the electrical current density be 0.5 A/cm² or more. It is more preferable that the electrical current density be 1.5 A/cm² or more or even more preferably 2.5 A/cm² or more.

Although there is no particular limitation on the upper limit of the electrical current density, it is preferable that the electrical current density be 20 A/cm² or less or more preferably 10 A/cm² or less.

Although there is no particular limitation on the conditions applied for electrolytic etching other than the electrical current density, for example, it is preferable that the time during which etching in the first etching process is performed be 10 seconds or more. In addition, it is preferable that the time during which etching in the first etching process is performed be 60 seconds or less.

### <Second etching process>

After the first etching process described above has been performed, etching in which the steel is brought into contact with an acidic solution having a pH of less than 7.0 is performed in the second etching process. Specifically, it is preferable that the steel be immersed in the acidic solution, washed thereafter with water, and dried.

The pH(25°C) of the acidic solution is less than 7.0. It is preferable that the acidic solution have a pH of 1.0 or less.

Although there is no particular limitation on the acidic solution, examples of the acidic solution which may be used include an etching solution for steel materials ("Metal Data Book, revised 4th edition", edited by the Japan Institute of Metals, published by Maruzen (2004)), nital (nitric acid: 1.5 ml, alcohol (at least one of methanol, ethanol, and amyl alcohol): 100 ml), picral (picric acid: 4 g, alcohol (methanol and/or ethanol): 100 ml), sodium picrate (picric acid: 2 g, sodium hydroxide: 25 g, water 100 ml), sodium hydroxide (aqueous solution containing 10 mass% of sodium hydroxide: 20 ml, hydrogen peroxide: 10 ml), and an etching solution containing 1 g to 4 g of potassium ferricyanide, 10 g of sodium hydroxide, and 100 ml of water.

In addition, examples of the acidic solution also include an etching solution containing 6.3 g of benzoic anhydride, 20 g of sodium hydroxide, and 100 ml of water and an etching solution containing 5 g of meta-nitrobenzene sulfonic acid and an alcohol solution.

In addition, examples of the acidic solution also include hydrochloric acid-picric acid (hydrochloric acid: 5 ml, picric acid: 1 g, alcohol (methanol and/or ethanol): 100 g) and an etching solution containing 10 ml of ortho-nitrophenol saturated in methanol and 20 ml of hydrochloric acid contained in amyl alcohol in an amount of 20 vol%.

In addition, examples of the acidic solution also include a 4 vol% glyceryl trinitrate solution, a 3 vol% to 4 vol% nitric acid alcohol solution (or aqueous solution), and an etching solution containing 5 g of ferric chloride, 50 ml of hydrochloric acid, and 100 ml of water.

In addition, examples of the acidic solution also include an etching solution containing 10 ml of nitric acid, 20 ml of hydrochloric acid, 20 ml of glycerin, and 10 ml of a hydrogen peroxide solution, an etching solution containing 10 g of ferric chloride, 30 ml of hydrochloric acid, and 120 ml of water, an etching solution containing 30 ml of hydrochloric acid and 10 ml of nitric acid, an etching solution containing saturated ferric chloride-hydrochloric acid and nitric acid, an etching solution containing 4 g of copper sulfate, 20 ml of hydrochloric acid, and 20 ml of water, an etching solution containing 5 g of copper sulfate, 100 ml of hydrochloric acid, 100 ml of ethanol, and 100 ml of water, an etching solution containing 10 ml of nitric acid, 20 ml to 30 ml of hydrochloric acid, and 20 ml to 30 ml of glycerin, an etching solution containing 10 g of potassium ferricyanide, 10 g of potassium hydroxide, and 100 ml of water, an etching solution containing 10 ml of hydrochloric acid, 3 ml of nitric acid, and 100 ml of methanol, an etching solution containing 20 ml of hydrochloric acid, 15 ml of water, 65 ml of ethanol, and 1 g of copper sulfate, a potassium ferricyanide neutral aqueous solution, an etching solution containing 30 ml of hydrochloric acid, 10 ml of nitric acid, and copper dichloride added to saturation, an etching solution containing 4 g of potassium permanganate, 1 g of sodium hydroxide, and 100 ml of water, an etching solution containing 10 g of potassium ferricyanide, 0.8 g of sodium hydroxide, and 100 ml of water, an etching solution containing 10 g of copper dichloride, 40 g of magnesium chloride, 20 ml of hydrochloric acid, and 1000 ml of ethanol, an etching solution containing 1 g of copper dichloride, 4 g of magnesium chloride, 1 ml of hydrochloric acid, 20 ml of water, and 100 ml of ethanol, and an etching solution containing 5 g of copper dichloride, 40 ml of hydrochloric acid, 30 ml of water, and 25 ml of alcohol.

In particular, considering the fact that steel or stainless steel having a microstructure including martensite and ferrite is preferably used as the steel which is subjected to etching in the present invention, to perform analysis on the microstructure with higher accuracy, it is preferable that the acidic solution contain at least one of hydrochloric acid, nitric acid, and picric acid. Examples of such an acidic solution include aqua regia (nitric acid: 15 ml, hydrochloric acid: 45 ml) and Vilella's reagent (ethanol: 50 ml, picric acid: 2 g, hydrochloric acid: 5 ml).

Although there is no particular limitation on the conditions applied for etching in which the steel is brought into contact with the acidic solution, for example, it is preferable that the time during which etching in the second etching process is performed be 10 seconds to 90 seconds.

By performing optical microscopic observation on the steel which is obtained by using the method for etching steel according to the present invention as described above and which is used as a sample, it is possible to analyze the microstructure of the steel with high accuracy.

For example, in the case where the steel has a microstructure including martensite and ferrite, since the whole surface of martensite is etched, contrast is less likely to be evident within martensite, which results in an improvement in the accuracy when the ferrite phase fraction is calculated by performing image analysis. Therefore, it is possible to determine the ferrite phase fraction of the steel with high accuracy and ease.

In addition, in the present invention, a method for preparing a sample for optical microscopic observation in which the steel obtained by using the method for etching steel according to the present invention described above is used as a sample for observation utilizing an optical microscope is also provided.

In addition, in the present invention, a solution set for etching steel including a basic solution and an acidic solution which is used in the method for etching steel according to the present invention described above is also provided.

Moreover, in the present invention, an etching apparatus including the solution set for etching steel described above and electrodes is also provided.

Regarding the etching apparatus, since it is preferable that constant electrical current be stably supplied when energizing is performed, a device such as a galvanostat may be used, and it is preferable that the magnitude of the electrical current be constant. In addition, it is preferable that the electrodes be made of stainless steel to inhibit the electrodes from being corroded in a basic solution or rust from occurring in the electrodes during storage.

### EXAMPLES

Hereafter, the present invention will be further described in accordance with examples. Here, the present invention is not limited to the examples below.

Molten steels having the chemical compositions given in Table 1 were prepared by using a vacuum high-frequency melting furnace, and ingots having a weight of 50 kg were manufactured. Such ingots were heated at a temperature of 1250°C for one hour and subjected to hot rolling so as to be made into steel plates having a thickness of 15 mm. Such steel plates were subjected to water quenching after having been heated at a temperature of 960°C for 20 minutes. The quenched steel plates were subjected to tempering in which heating was performed at a temperature of 600°C for 30 minutes and then subjected to natural cooling. Subsequently, small samples for microstructure investigation were taken in such a manner that a surface parallel to the rolling direction and the plate thickness direction was the observation surface. The small samples were embedded in a resin and subjected to mirror polishing. The mirror-polished samples were subjected to etching under the three conditions described below.

**[Table 1]**

| Chemical Composition (mass%) | | | | | | | | | | | | | |
|---|---|---|---|---|---|---|---|---|---|---|---|---|---|
| C | Si | Mn | P | S | Cu | Ni | Cr | Total-Al | Mo | V | N | O | W |
| 0.03 | 0.25 | 0.30 | 0.015 | 0.0015 | 1.0 | 3.8 | 16.6 | 0.03 | 2.5 | 0.04 | 0.05 | 0.002 | 0.9 |

**The remainder of the chemical composition which differs from the constituents described above is Fe and incidental impurities.**

### [Etching condition (1) - Example 1 of the present invention]

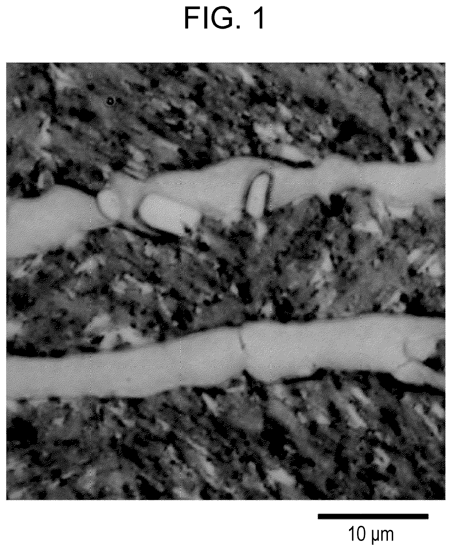

Electrolytic etching was performed in a 25 mass% KOH aqueous solution by using an anodic polarization method with an electrical current density of 3.0 A/cm² for 35 seconds. After the etched sample had been washed with water and dried, the dried sample was immersed in Vilella's reagent (ethanol: 50 ml, picric acid: 2 g, hydrochloric acid: 5 ml) for 30 seconds, washed with water again, and dried. Fig. 1 shows an example of an optical micrograph of the microstructure of the obtained sample at a magnification of 1000 times.

### [Etching condition (2) - Comparative example 1: mixture solution of aqua regia and glycerin]

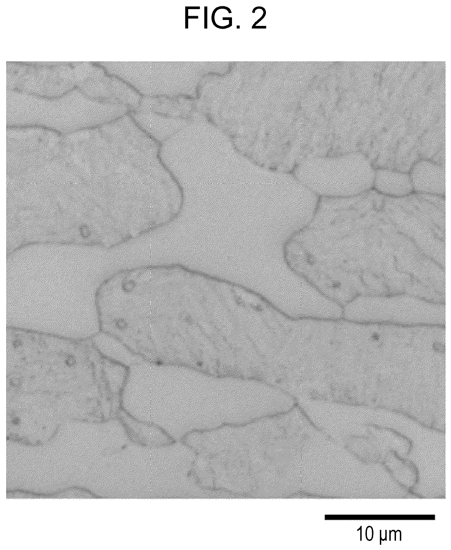

The sample was immersed in a mixture solution of aqua regia (mixture of 15 ml of nitric acid and 45 ml of hydrochloric acid) and 30 ml of glycerin for 30 seconds, washed with water, and dried. Fig. 2 shows an example of an optical micrograph of the microstructure of the obtained sample at a magnification of 1000 times.

### [Etching condition (3) - Comparative example 2: Vilella's reagent]

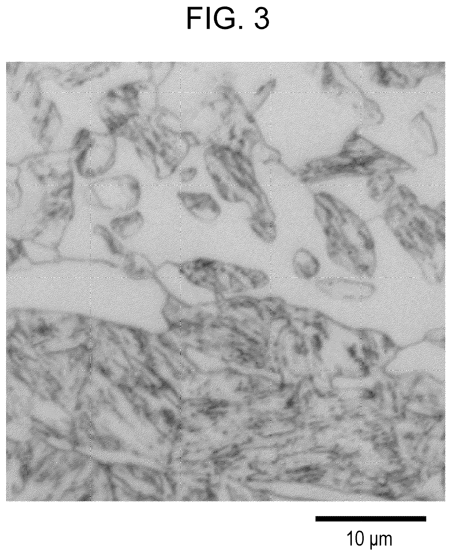

The sample was immersed in Vilella's reagent (ethanol: 50 ml, picric acid 2 g, hydrochloric acid: 5 ml) for 30 seconds, washed with water, and dried. Fig. 3 shows an example of an optical micrograph of the microstructure of the obtained sample at a magnification of 1000 times.

### [Etching condition (4) - Example 2 of the present invention]

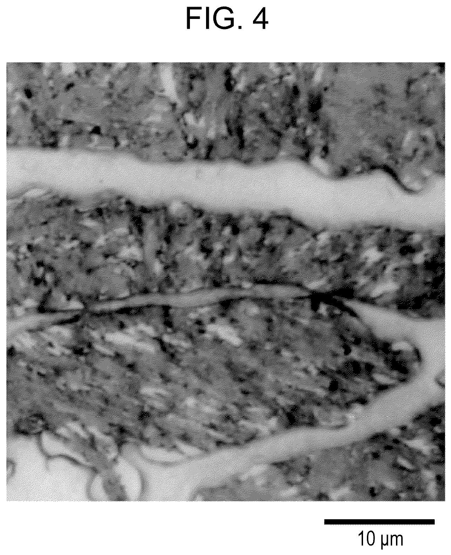

Electrolytic etching was performed in a 25 mass% NaOH aqueous solution by using an anodic polarization method with an electrical current density of 3.0 A/cm² for 35 seconds. After the etched sample had been washed with water and dried, the dried sample was immersed in Vilella's reagent (ethanol: 50 ml, picric acid: 2 g, hydrochloric acid: 5 ml) for 30 seconds, washed with water again, and dried. Fig. 4 shows an example of an optical micrograph of the microstructure of the obtained sample at a magnification of 1000 times.

In Fig. 1 and Fig. 4, that is, in the case of the examples of the present invention, regions of martensite, which had a dark appearance in terms of micrograph contrast, were generally dark and had large contrast differences with respect to the regions of ferrite, which had a light appearance in terms of micrograph contrast.

In the case of Comparative example 1 shown in Fig. 2 where a mixture solution of aqua regia and glycerin was used, since regions of martensite, which had a darker appearance in terms of micrograph contrast, were not etched much, there is only a small contrast difference between martensite and ferrite.

In the case of such a micrograph, since there is only a small contrast difference between martensite and ferrite, it is not possible to distinguish with high accuracy between martensite and ferrite by performing image analysis.

In addition, Fig. 3 shows the optical micrograph of Comparative example 2 in which Vilella's reagent was used, and regions which had a darker appearance in terms of micrograph contrast correspond to martensite. Portions of martensite had a light appearance in terms of the micrograph contrast in such a manner that the lightness of such portions were equivalent to that of ferrite. Since portions of martensite had lightness equivalent to that of ferrite, it is not possible to distinguish with high accuracy between martensite and ferrite by performing image analysis.

On the other hand, in the case of the examples of the present invention, since there was a large contrast difference between martensite and ferrite, it is possible to distinguish with high accuracy between martensite and ferrite by performing image analysis.

## Claims

1. A method for etching steel, the method comprising:
a first etching process of performing electrolytic etching on steel in a basic solution having a pH of more than 7.0; and
a second etching process of bringing the steel, which has been subjected to the first etching process, into contact with an acidic solution having a pH of less than 7.0.

2. The method for etching steel according to Claim 1,
wherein the basic solution has a pH of 13.0 or more.

3. The method for etching steel according to Claim 1 or 2, wherein the basic solution is a KOH aqueous solution or a NaOH aqueous solution.

4. The method for etching steel according to any one of Claims 1 to 3, wherein the electrolytic etching is performed with an electrical current density of 0.5 A/cm² or more.

5. The method for etching steel according to any one of Claims 1 to 4, wherein the steel has a microstructure including martensite and ferrite.

6. A method for preparing a sample for optical microscopic observation, wherein the steel which has been obtained by using the method for etching steel according to any one of Claims 1 to 5 is used as the sample for optical microscopic observation.

7. A solution set for etching steel, the solution set comprising:
a basic solution having a pH of more than 7.0 for performing electrolytic etching on steel; and
an acidic solution having a pH of less than 7.0 for performing etching on the steel.

8. The solution set for etching steel according to Claim 7, wherein the basic solution has a pH of 13.0 or more.

9. The solution set for etching steel according to Claim 7 or 8, wherein the basic solution is a KOH aqueous solution or a NaOH aqueous solution.

10. The solution set for etching steel according to any one of Claims 7 to 9, wherein the steel has a microstructure including martensite and ferrite.

11. An etching apparatus comprising:
the solution set for etching steel according to any one of Claims 7 to 10; and
electrodes.
